# EUROPÄISCHE PATENTANMELDUNG

(11) **EP 4 825 849 A1**
(43) Veröffentlichungstag der Anmeldung: **21.10.2026**
(21) Anmeldenummer: 25171286.5
(22) Anmeldetag: 17.04.2025
(51) Int. Cl.: C07D 307/24

(54) **VERFAHREN ZUR HERSTELLUNG VON CIS-4-AMINOTETRAHYDROFURAN-2-CARBONSÄUREESTERN**

(71) Anmelder: Bayer Aktiengesellschaft, 51373 Leverkusen (DE)
(72) Erfinder: Die Erfindernennung liegt noch nicht vor
(74) Vertreter: BIP Patents

(57) **Zusammenfassung**

Die vorliegende Erfindung betrifft ein neues Verfahren zur Herstellung der Salze von cis-4-Aminotetrahydrofuran-2-carbonsäureester der allgemeinen Formel (I).

## Beschreibung

Die vorliegende Erfindung betrifft ein neues Verfahren zur Herstellung der Salze von cis-4-Aminotetrahydrofuran-2-carbonsäureestern der allgemeinen Formel (**I**).

Cis-4-Aminotetrahydrofuran-2-carbonsäuremethylester Hydrochlorid (CAS 1304126-28-0) der allgemeinen Formel **(I)** (mit R¹=Methyl) ist ein wichtiger Baustein zur Synthese von Pflanzenschutzmitteln (WO 2012/130798, WO 2021/170464).

Die erste publizierte Syntheseroute ist von Walker et al. (Synthesis 2011, 7, 1113-1119). Es wird 4-(tert-Butoxycarbonylamino)furan-2-carbonsäuremethylester (CAS 1170719-58-0, Wolter et al., Org. Lett. 2009, 11, 2804) als Vorstufe verwendet. 4-(tert-Butoxycarbonylamino)furan-2-carbonsäuremethylester ist allerdings aufgrund der hohen Kosten der Reagenzien zur Herstellung, der geringen Ausbeuten, Sicherheitsrisiken und Phosphatabfällen im großtechnischen Maßstab nicht wirtschaftlich herstellbar. Beispielsweise sind dafür eine Lithiierung mit sec-Butyllithium (34% Ausbeute) und eine Curtius Umlagerung mit DPPA (Diphenylphosphonazid) notwendig. Daher ist diese Route zur Herstellung von cis-4-Aminotetrahydrofuran-2-carbonsäureestern im industriellen Maßstab nicht machbar.

Auch die Synthese über 4-Bromfuran-2-carbonsäuremethylester (CAS 58235-80-6, WO 2021/170464) ist aufgrund der Bromierung und der anschließenden ineffizienten Kupplung mit tert-Butylcarbamat (25% Ausbeute, WO 2021/170464) kostenintensiv und führt zu großen Mengen an Abfällen.

Ein Verfahren zur Synthese der Verbindungen der allgemeinen Formel **(I)** in Form eines Salzes durch Hydrierung von Verbindungen der allgemeinen Formel **(II)** wird in WO 2023/161204 dargestellt, wobei zur Kristallisation und Isolierung von **(I)** ein Lösungsmittelgemisch aus Methanol und Toluol verwendet wird.

Im Lichte des zuvor beschriebenen Standes der Technik liegt der vorliegenden Erfindung die Aufgabe zugrunde, ein Verfahren zur Herstellung von cis-4-Aminotetrahydrofuran-2-carbonsäureestern zu finden, das kostengünstig ist und großtechnisch eingesetzt werden kann. Auch ist es erstrebenswert, diese Verbindungen umweltfreundlich, in hoher Ausbeute und in hoher Reinheit zu erhalten, so dass sie keiner weiteren komplexen Aufreinigung unterzogen werden müssen, wodurch dann auch die entsprechenden Pflanzenschutzmittel im großtechnischen Maßstab hergestellt werden können.

Die zuvor beschriebene Aufgabe - einfache, kostengünstige und großtechnische Herstellung - wird gelöst durch ein Verfahren zur Herstellung von Verbindungen der allgemeinen Formel **(I)** in Form eines Salzes 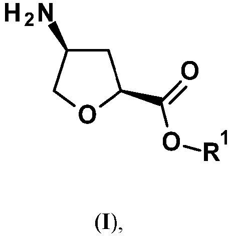 worin
- R¹: für (C₁-C₆)Alkyl oder (C₃-C₆)Cycloalkyl steht,
dadurch gekennzeichnet, dass in einem ersten Schritt Verbindungen der allgemeinen Formel **(II)** in Form eines Salzes 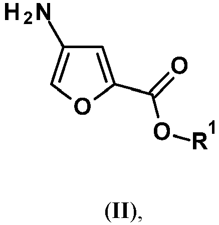 in Gegenwart von Wasserstoff, einem Rhodium-Katalysator in einem Lösungsmittel zu Verbindungen der allgemeinen Formel **(I)** umgesetzt werden und dann in einem zweiten Schritt in einem Lösungsmittel R¹OH oder einem etherischen Lösungsmittel oder einem Ester-Lösungsmittel oder einem Carbonatlösungsmittel oder einer Mischung der genannten Lösungsmittel in Form eines Salzes kristallisiert werden.

Bevorzugt wird die Reaktion mit einem Rh/Alox oder Rh/C Katalysator durchgeführt.

Bevorzugt wird die Reaktion im Lösungsmittel R¹OH/tert.-Butylmethylether durchgeführt.

In einer weiteren Ausführungsform wird dem Reaktionsgemisch eine Säure hinzugefügt.

Bevorzugte Restedefinitionen für die Verbindungen der allgemeinen Formeln **(I)** und **(II)** sind die folgenden:
- R¹: steht für (C₁-C₆)Alkyl.

Besonders bevorzugte Restedefinitionen für die Verbindungen der allgemeinen Formeln **(I)** und **(II)** sind die folgenden:
- R¹: steht für CH₃.

Die Reaktion zur Herstellung von Verbindungen der Formel (I) ist im Schema 1 dargestellt.

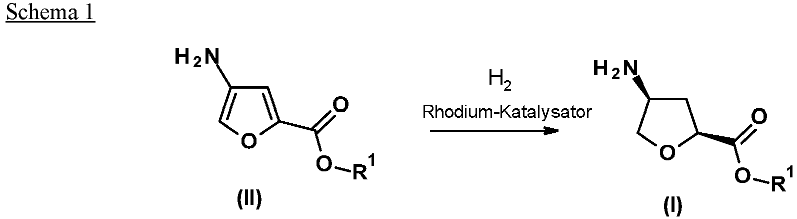

Die Verbindungen der Formel **(II)** in Form eines Salzes reagieren in Gegenwart von Wasserstoff, einem Rhodium Katalysator in einem Lösungsmittel zu Verbindungen der allgemeinen Formel (**I**).

Der Druck bei der Reaktion beträgt 1 bis 100 bar, bevorzugt 5 bis 50 bar, besonders bevorzugt 20 bis 50 bar.

Die Temperatur bei der Reaktion beträgt 0°C bis 100 °C, bevorzugt 10°C bis 80°C, besonders bevorzugt 20 bis 40°C.

Als Katalysatoren können die folgenden Rhodium-Katalysatoren oder gemischte Katalysatoren bestehend aus Rhodium und einem oder mehreren weiteren Metallen eingesetzt werden, beispielsweise: Rh/C, Rh/Alox, Rh+Pd/C, Rh+Pt/C, Rh+Cu/C, Rh+Ru/C; bevorzugt: Rh/C, Rh/Alox.

Die Katalysatoren werden mit einer Edelmetall-Beladung von 0,5-10% Edelmetall auf dem Trägermaterial eingesetzt; bevorzugt werden 1-5% Edelmetall auf dem Trägermaterial aufgebracht.

Die Katalysatoren werden mit einer Menge von 0,1-10 Gew.-% in Bezug auf die Verbindungen der Formel **(II)** eingesetzt; bevorzugt werden 0,5-5 Gew.-% eingesetzt.

Die Menge der eingesetzten Katalysatoren wird auf die Trockenmasse der Katalysatoren berechnet. Die Katalysatoren können trocken oder wasserfeucht eingesetzt werden. Wasserfeuchte Katalysatoren können vor der Reaktion mit trockenem Lösungsmittel unter Inertgas-Atmosphäre gewaschen werden, um anhaftendes Wasser zu entfernen. Alternativ kann das Wasser durch azeotrope Destillation mit einem geeigneten Lösungsmittel entfernt werden. Für die azeotrope Destillation kann beispielsweise Toluol eingesetzt werden.

Der Katalysator kann einmal oder bevorzugt auch mehrfach wiederverwendet bzw. eingesetzt werden. Eine Reaktivierung des Katalysators kann, beispielsweise durch geeignete Wäschen, vorteilhaft für dessen Wiederverwendung sein, beispielsweise durch Wäschen mit Methanol oder saurem Methanol.

Die Verbindungen der Formel **(II)** werden als Salze ggf. mit einer Säure eingesetzt. Dem Reaktionsgemisch kann zusätzlich eine weitere Säure oder ein Säuregemisch hinzugefügt werden. Bevorzugt werden folgende Säuren zur Salzbildung mit Verbindungen der Formel **(II)** und/oder als Zusatz eingesetzt: HCl, H₂SO₄, MsOH, TfOH, TFA. Besonders bevorzugt ist HCl.

Das molare Verhältnis der Säure zur Verbindung der Formel **(II)** liegt bei 0-100%, bevorzugt bei 0-10%.

Geeignete Lösungsmittel und Lösungsmittelgemische sind z.B.: R¹OH, R'OH/Toluol, R'OH/etherische Lösungsmittel, R¹OH/Ester-Lösungsmittel oder R¹OH/Carbonat-Lösungsmittel. Bevorzugt ist R¹OH und R¹OH/tert.-Butylmethylether.

Fallen die Verbindungen der allgemeinen Formel **(I)** in Form ihrer Salze an, z.B. als Hydrochlorid, kann die salzfreie Form gewonnen werden, indem das Salz mit einer Base, z.B. Triethylamin, behandelt wird.

Die Verbindungen der allgemeinen Formel **(I)** in Form ihrer Salze können in einem zweiten Schritt durch Kristallisation aus geeigneten Lösungsmitteln und Lösungsmittelgemischen in hoher Reinheit und Ausbeute erhalten werden. Geeignete Lösungsmittel und Lösungsmittelgemische sind z.B.: R¹OH, etherische Lösungsmittel, Ester-Lösungsmittel, Carbonatlösungsmittel, R'OH/etherische Lösungsmittel, R¹OH/Ester-Lösungsmittel oder R¹OH/Carbonat-Lösungsmittel. Etherische Lösungsmittel sind z.B. 2-Methyltetrahydrofuran, 4-Methyltetrahydropyran, Cyclopentylmethylether, Diisopropylether, tert.-Amylmethylether und tert.-Butylmethylether. Ester-Lösungsmittel sind z.B: Ethylacetat, Isopropylacetat, Amylacetat, Isoamylacetat. Carbonat-Lösungsmittel sind z.B: Dimethylcarbonat oder Diethylcarbonat. Bevorzugt ist R¹OH/tert.-Butylmethylether.

Verbindungen der allgemeinen Formel **(II)** werden gemäß WO 2022/018057 hergestellt.

### Erläuterung des Verfahrens

### Beispiele

Die vorliegende Erfindung wird anhand der nachfolgenden Beispiele näher erläutert, ohne die Erfindung dabei auf diese einzuschränken.

### Messverfahren

Die Produkte wurden mittels ¹H-NMR Spektroskopie und/oder GC/MS (Gas Chromatography Mass Spectrometry) charakterisiert.

Die NMR-Spektren wurden mit einem Bruker AV III 600 gemessen.

Die GC/MS Proben wurden mit einem Shimadzu GCMS-QP-2010-Ultra gemessen, das mit einem zusätzlichen FID (Flammen-Ionisations-Detektor) gekoppelt ist. Dazu wurden die Proben zunächst eingedampft und dann 1-10 mg trockene Probe zur Silylierung mit 250 µL N-Methyl-N-trimethylsilyltrifluoracetamid versetzt. Nach 1-5 Minuten Reaktionszeit wurden die Proben mit 1 ml Acetonitril verdünnt und gemessen.

Die quantitativen GC-Bestimmung wurden auf einer GC Agilent HB7890 B-Serie gemessen. Die Technik beruht auf GC mit FID Detektion, einer RTX-5 Amin Säule und mit interner Standard Auswertung (Interner Standard: Diethylphthalat). Die Proben, Referenzstandard, sowie interner Standard werden in Methanol gelöst.

### Herstellung von rac-cis-4-Aminotetrahydrofuran-2-carbonsäuremethylester Hydrochlorid

### Vergleichsbeispiel mit Toluol als Co-Solvens zur Kristallisation des Zielprodukts

### Beispiel 1

Eine Lösung von 50 g 4-Aminofuran-2-carbonsäuremethylester Hydrochlorid (282 mmol) in 250 g Methanol wurde in einem 600 ml Autoklaven mit 2,5 g eines 5% Rh/C Katalysators (trocken, 1,22 mmol Rh) versetzt und der Autoklav dreimal mit 5 bar Argon gespült. Die Mischung wurde unter 50 bar Wasserstoff 16 h bei 50°C und 600 U/min gerührt. Der Autoklav wurde abgekühlt, entspannt und die Mischung über eine Nutsche filtriert. Das Filtrat wurde unter reduziertem Druck auf 123 g eingeengt und es wurden 250 ml Toluol bei 0°C zugetropft. Die Mischung wurde bei 0°C 30 min gerührt und der Niederschlag abgesaugt, 2x mit 15 ml Toluol gewaschen und unter reduziertem Druck bei 50°C getrocknet.
Ausbeute: 36,2 g (66% der Theorie) der Zielverbindung
Reinheit (quant. NMR): 93,5% der Zielverbindung
¹H-NMR (DMSO-d6, 600 MHz): 1,95-2,04 (1H, m), 2,59-2,67 (1H, m), 3,70 (3H, s), 3,75-3,85 (2H, m), 3,9-3,97 (1H, m), 4,45-4,52 (1H, t), 8,2-8,5 (3H, br) ppm.
GC/MS (m/z): 217 [M+TMS], 202, 187, 172, 158, 142, 128, 116, 100, 89, 73, 59, 54

### Beispiele mit tert.-Butylmethylether als Co-Solvens zur Kristallisation des Zielprodukts

### Beispiel 2

Eine Mischung aus 20 g 4-Aminofuran-2-carbonsäuremethylester Hydrochlorid (Gehalt: 94,7%, 107 mmol) und 100 g Methanol wurde in einem 600 ml Autoklaven mit 1 g eines 5% Rh/C Katalysators versetzt und der Autoklav dreimal mit 5 bar Argon gespült. Die Mischung wurde unter 50 bar Wasserstoff 2 h bei 50°C und 600 U/min gerührt. Der Autoklav wurde abgekühlt, entspannt und die Mischung über eine Nutsche filtriert. Das Filtrat wurde unter reduziertem Druck auf 40 g eingeengt, auf 0 °C gekühlt und nach Rühren bei 0 °C für 30 min dann tropfenweise mit 80 ml einer Mischung aus 85% tert.-Butylmethylether und 15% Methanol versetzt und weitere 30 min bei 0°C gerührt. Der entstandene Niederschlag wurde über eine Nutsche abgesaugt und 2 x mit einer Mischung aus 85% tert.-Butylmethylether und 15% Methanol gewaschen und dann unter reduziertem Druck getrocknet.
Ausbeute: 14,88 g (74,7% der Theorie) der Zielverbindung
Reinheit (quant. GC): 97,2% der Zielverbindung

### Beispiel 3

Eine Mischung aus 17,5 g 4-Aminofuran-2-carbonsäuremethylester Hydrochlorid (Gehalt: 94,7%, 93,3 mmol) und 87,5 g Methanol wurde in einem 600 ml Autoklaven mit 0,875 g eines 5% Rh/C Katalysators versetzt und der Autoklav dreimal mit 5 bar Argon gespült. Die Mischung wurde unter 30 bar Wasserstoff 5 h bei 30°C und 600 U/min gerührt. Das Filtrat wurde unter reduziertem Druck auf 36 g eingeengt, bei 0°C 30 min gerührt und dann nach tropfenweiser Zugabe von 70 ml einer Mischung aus 85% tert.-Butylmethylether und 15% Methanol weitere 30 min bei 0°C gerührt. Der entstandene Niederschlag wurde über eine Nutsche abgesaugt und 2 x mit einer Mischung aus 85% tert.-Butylmethylether und 15% Methanol gewaschen und dann unter reduziertem Druck getrocknet.
Ausbeute: 13,65 g (76,5 % der Theorie) der Zielverbindung
Reinheit (quant. GC): 94,9% der Zielverbindung

### Beispiel 4

Eine Mischung aus 50 g 4-Aminofuran-2-carbonsäuremethylester Hydrochlorid (Gehalt: 94,7%, 267 mmol) und 350 ml Methanol wurde in einem 600 ml Autoklaven mit 3 g eines 5% Rh/Alox Katalysators versetzt und der Autoklav dreimal mit 5 bar Argon gespült. Die Mischung wurde unter 30 bar Wasserstoff 3,5 h bei 30°C und 600 U/min gerührt. Der Autoklav wurde abgekühlt, entspannt und die Mischung über eine Nutsche filtriert. Das Filtrat wurde unter reduziertem Druck auf 87 g eingeengt, auf 0 °C gekühlt sowie bei 0°C 30 min gerührt und dann tropfenweise mit einer Mischung aus 170 ml tert.-Butylmethylether und 30 ml Methanol versetzt und weitere 30 min bei 0°C gerührt. Der entstandene Niederschlag wurde über eine Nutsche abgesaugt und 2 x mit einer Mischung aus 85% tert.-Butylmethylether und 15% Methanol gewaschen und dann unter reduziertem Druck getrocknet.
Ausbeute: 37,4 g (76,1 % der Theorie) der Zielverbindung
Reinheit (quant. GC): 98,6% der Zielverbindung

### Beispiel 5

Eine Mischung aus 50 g 4-Aminofuran-2-carbonsäuremethylester Hydrochlorid (Gehalt: 94,7%, 267 mmol), 280 ml Methanol und 70 ml tert.-Butylmethylether wurde in einem 600 ml Autoklaven mit 3 g eines 5% Rh/Alox Katalysators versetzt und der Autoklav dreimal mit 5 bar Argon gespült. Die Mischung wurde unter 30 bar Wasserstoff 4 h bei 30°C und 600 U/min gerührt. Der Autoklav wurde abgekühlt, entspannt und die Mischung über eine Nutsche filtriert. Das Filtrat wurde unter reduziertem Druck auf 73 g eingeengt, bei 40-50°C 30 min gerührt und dann eine Mischung aus 170 ml tert.-Butylmethylether und 30 ml Methanol in 30 min zugegeben. Die Mischung wurde auf 0°C abgekühlt und weitere 30 min bei 0°C gerührt. Der entstandene Niederschlag wurde über eine Nutsche abgesaugt und 2 x mit einer Mischung aus 80% tert.-Butylmethylether und 20% Methanol gewaschen und dann unter reduziertem Druck getrocknet.
Ausbeute: 39,17 g (79,3 % der Theorie) der Zielverbindung
Reinheit (quant. GC): 98,0% der Zielverbindung.

## Patentansprüche

1. Verfahren zur Kristallisation von Verbindungen der allgemeinen Formel (I) in Form eines Salzes 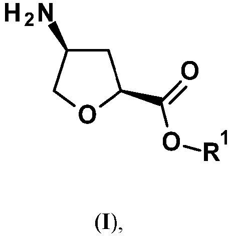 worin
R¹ für (C₁-C₆)Alkyl oder (C₃-C₆)Cycloalkyl steht,
**dadurch gekennzeichnet, dass** die Verbindungen der allgemeinen Formel (I) in einem Lösungsmittel R¹OH, einem etherischen Lösungsmittel, einem Ester-Lösungsmittel, einem Carbonatlösungsmittel oder einer Mischung der genannten Lösungsmittel kristallisiert werden.

2. Verfahren zur Herstellung von Verbindungen der allgemeinen Formel (I) in Form eines Salzes 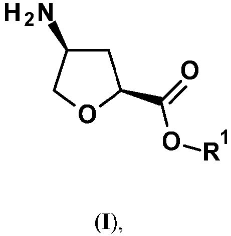 worin
R¹ für (C₁-C₆)Alkyl oder (C₃-C₆)Cycloalkyl steht,
**dadurch gekennzeichnet, dass** Verbindungen der allgemeinen Formel (II) in Form eines Salzes 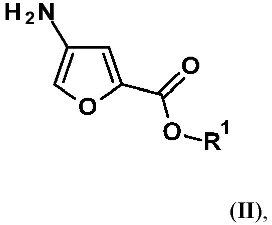 in Gegenwart von Wasserstoff, einem Rhodium-Katalysator in einem Lösungsmittel zu Verbindungen der allgemeinen Formel **(I)** umgesetzt werden und dann in einem zweiten Schritt in einem Lösungsmittel R¹OH, einem etherischen Lösungsmittel, einem Ester-Lösungsmittel, einem Carbonatlösungsmittel oder einer Mischung der genannten Lösungsmittel in Form eines Salzes kristallisiert werden.

3. Verfahren nach Anspruch 1 oder 2, **dadurch gekennzeichnet, dass** die Restedefinitionen für die Verbindungen der allgemeinen Formeln **(I)** und **(II)** die folgenden sind:
R¹ steht für (C₁-C₆)Alkyl.

4. Verfahren nach Anspruch 3, **dadurch gekennzeichnet, dass** die Restedefinitionen für die Verbindungen der allgemeinen Formeln **(I)** und **(II)** die folgenden sind:
R¹ steht für CH₃.

5. Verfahren nach einem der Ansprüche 2 bis 4, **dadurch gekennzeichnet, dass** dem Reaktionsgemisch eine Säure hinzugefügt wird.

6. Verfahren nach Anspruch 5, **dadurch gekennzeichnet, dass** zum Reaktionsgemisch eine weitere Säure oder Säuregemisch hinzugefügt wird.

7. Verfahren nach Anspruch 5 oder 6, **dadurch gekennzeichnet, dass** zum Reaktionsgemisch HCl als Säure hinzugefügt wird.

8. Verfahren nach einem der Ansprüche 1 bis 7, **dadurch gekennzeichnet, dass** R¹ Methyl ist.

9. Verfahren nach einem der Ansprüche 2 bis 8, **dadurch gekennzeichnet, dass** 0.1 bis 10 Gew.% Katalysator (Trockengewicht) eingesetzt werden bezogen auf Verbindungen der Formel **(II).**

10. Verfahren nach einem der Ansprüche 2 bis 9, **dadurch gekennzeichnet, dass** als Katalysator Rh/Alox verwendet wird.

11. Verfahren nach einem der Ansprüche 1, 2 oder 8, **dadurch gekennzeichnet, dass** R¹OH/tert.-Butylmethylether zur Kristallisation der Verbindungen der allgemeinen Formel **(I)** eingesetzt wird.
